# EUROPÄISCHE PATENTANMELDUNG

(11) **EP 2 018 849 A2**
(43) Veröffentlichungstag der Anmeldung: **28.01.2009**
(21) Anmeldenummer: 08013148.5
(22) Anmeldetag: 22.07.2008
(51) Int. Cl.: A61K 8/67, A61Q 19/00

(54) **Kosmetisches Verfahren zur Beeinflussung der Haut**

(30) Priorität: 26.07.2007 DE 102007034957
(71) Anmelder: Hönscher-Sickert, Monika, 46286 Dorsten (DE)
(72) Erfinder: Hönscher-Sickert, Monika, 46286 Dorsten (DE)
(74) Vertreter: Stenger, Watzke & Ring

(57) **Zusammenfassung**

Die Erfindung betrifft ein kosmetisches Verfahren zur Beeinflussung der Haut mit folgenden Verfahrensschritten:
- Feststellen der Hautsituation durch Bestimmung des Pflegezustandes der Haut, des Alters und/oder der Energie- und Belastungssituation der Person;
- Feststellen der Mangelsituation der Haut in Abhängigkeit der zuvor festgestellten Hautsituation;
- Festlegen eines Behandlungsplans in Abhängigkeit der zuvor festgestellten Mangelsituation der Haut, umfassend die Aufnahme von Nahrungsergänzungspräparaten zur inneren Behandlung der festgestellten Mangelsituation der Haut und eine äußere Applikation von nährstoffhaltigen Verbindungen zur gleichzeitigen ergänzenden äußeren Behandlung der festgestellten Mangelsituation der Haut.

## Beschreibung

Die vorliegende Patentanmeldung betrifft ein kosmetisches Verfahren zur Beeinflussung der Haut. Insbesondere betrifft die vorliegende Erfindung ein kosmetisches Verfahren zur Verzögerung von Alterserscheinungen der Haut.

Die menschliche Haut ist ständig den unterschiedlichsten Alltagseinflüssen ausgesetzt, welche zu einer Belastung der Haut und damit zu vorzeitigen Alterserscheinungen führen können.

Durch die modernen Lebensgewohnheiten und Belastungen in der Arbeitswelt wird die menschliche Haut zusätzlich durch Stressfaktoren belastet. Stress- und Umweltbelastungen können zu Nährstoffmangel in der Haut führen. Dieser Nährstoffmangel führt zu spezifischen Mangelerscheinungen der Haut, welche wiederum mit spezifischen Alterserscheinungen einhergehen.

Die zunehmende Konservierung unserer Nahrung macht es vielfach unmöglich, unseren täglichen Bedarf zum Beispiel an Folsäure, Selen und Vitamin D über die Nahrungszufuhr abzudecken. Die zunehmenden Stress- und Umweltbelastungen wie Rauchen, Alkohol, Medikamente, Strahlenbelastung durch Mobiltelefone, Verkehr und Computerarbeit führen zu einem erhöhten Bedarf an Nährstoffen, den der moderne Mensch aber wegen der Konservierung der Nahrungsmittel in den meisten Fällen gar nicht mehr durch normale Nahrungsaufnahme decken kann. Ganzheitliche, die Gesundheit der Haut beeinträchtigende Funktionsstörungen sowie unreine, spröde, fahle und zu Entzündungen neigende Haut sind die Folge.

Zur Behandlung bzw. Bekämpfung dieser Symptome sind unterschiedliche Ansätze bekannt. Die bekannten Ansätze führen jedoch nur bedingt zu dem gewünschten Erfolg, also einer sichtbaren Verbesserung der Hautsituation bzw. Verminderung der Alterserscheinungen der Haut.

Es ist die Aufgabe der vorliegenden Patentanmeldung ein Verfahren zur Beeinflussung der Haut anzugeben, mit welchem die durch Stress- und Umweltbelastungen hervorgerufenen Mangelsituationen der Haut nachhaltig behoben werden können und so eine deutliche Verbesserung des Erscheinungsbildes der Haut erzielt wird.

Gelöst wird diese Aufgabe durch ein kosmetisches Verfahren zur Beeinflussung der Haut, aufweisend die Verfahrensschritte:
Feststellen der Hautsituation durch Bestimmung des Pflegezustandes der Haut, des Alters sowie der Energie- und Belastungssituation der Person;
Feststellen der Mangelsituation der Haut in Abhängigkeit der zuvor festgestellten Hautsituation;
Festlegen eines Behandlungsplans in Abhängigkeit der zuvor festgestellten Mangelsituation der Haut, umfassend die Aufnahme von Nahrungsergänzungspräparaten zur inneren Behandlung der festgestellten Mangelsituation der Haut und eine äußere Applikation von nährstoffhaltigen Verbindungen zur gleichzeitigen ergänzenden äußeren Behandlung der festgestellten Mangelsituation der Haut.

Es wurde festgestellt, dass bestimmte Energie- und Belastungssituationen von Personen mit bestimmten Mangelerscheinungen bzw. Mangelsituationen der Haut korrelieren. So führt beispielsweise erhöhter Stress zu einen Mangel an Vitaminen der B-Reihe wie beispielsweise B1, B2, B3, B5, B6 und B12. Darüber hinaus führt erhöhter Stress zu einem erhöhten Bedarf an Folsäure, Biotin, Cholin, Inosit und para-Aminobenzoesäure.

Ein Vitamin B Mangel kann sich in Haarausfall und spröder, schlecht heilender Haut äußern. Eine ausgewogene Zufuhr aller Vitamine der B-Gruppe ist notwendig, da die Langzeiteinnahme eines Monopräparates zu einer negativen Beeinflussung der Schilddrüsen- und Bauchspeicheldrüsenfunktion führen kann.

Darüber hinaus führt Stress zu einer erhöhten Anfälligkeit gegenüber Radikalen, so dass ein erhöhter Bedarf an Antioxidanzien in der Haut besteht. Diesem erhöhten Bedarf kann durch oligomere Procyandine oder Proanthocydine, wie sie aus Traubenkernextrakt oder Kiefernrindenextrakt gewonnen werden können, entgegengewirkt werden. Darüber hinaus dienen Zitronenbioflavonoide, Hesperidin, Vitamin C, Rutin und Quercetin zur Verringerung der Stress- und Radikalbelastung der Haut.

Oligomere Procyandine oder Proanthocydine besitzen eine ca. 20-fach stärkere antioxidative Wirkung im Vergleich zu Vitamin C und eine ca. 50-fach stärkere antioxidative Wirkung im Vergleich zu Vitamin E. Darüber hinaus besitzen diese Verbindungen eine hohe Bindegewebsaffinität und können Gefäße und das Collagen vor Brüchen schützen. Insbesondere Brüche im Collagen führen zu sichtbaren Alterserscheinungen der Haut.

Stress kann zu einer erhöhten Belastung der Darmflora, bis hin zu akuten Magendarmproblemen führen. Viele Nährstoffe werden jedoch vom Organismus nur dann vollständig genutzt, wenn sie über den Darm aufgenommen werden können. Voraussetzung hierfür ist jedoch eine gesunde und intakte Darmflora. Zur Verbesserung der stressbedingten Hautsituation wurde deshalb festgestellt, dass die innere Anwendung von Milchsäurebakterien wie Lactobacillus Acidophilus, Lactobacillus Plantarum, Lactobacillus Casei, Bifido-bacterium Longum, Streptococcus Thermophilus oder Lactobacillus Brevus zu einer deutlichen Verbesserung der stressbedingten Hautsituation führt.

Allgemein dient ein ausgeglichenes und gesundes Immunsystem ebenfalls der Beseitigung von stressbedingten Belastungs- und Mangelsituationen der Haut. Zur Verbesserung des Immunsystems und damit einhergehenden kosmetischen Behandlung von stressbedingten Hauterscheinungen können daher Vitamin A, Vitamin C, Vitamin E sowie Spurenelemente wie Zink und Selen das Immunsystem unterstützen. Auch die Einnahme von Pflanzenextraten wie Traubenkernextrakt, Heidelbeerextrakt, Grünteeextrakt, Milchdistelextrakt, Ginko Bilobaextrakt, Kudzuextrakt, Holunderbeerenextrakt oder rote Traubenextrakt können zur Unterstützung des Immunsystems zur kosmetischen Behandlung von stressbedingten Hauterscheinungen eingesetzt werden.

Darüber hinaus kann die Aufnahme von Verbindungen wie Co-Enzym Q10, alpha-Liponsäure, N-Acetyl-I-Cystein, L-Glutathion, Beta-Carotinoide, Katalase oder Taurin zur Unterstützung des Immunsystems im Rahmen der kosmetischen Behandlung von stressbedingten Hauterscheinungen dienen. Ebenfalls können weitere Pflanzenpräparate wie Kurkumaölpulver, grüne Teeblätter oder cat's claw eingesetzt werden.

Es wurde festgestellt, dass erhöhte Stressbelastung ebenfalls zu einem erhöhten Bedarf an Mineralien und Spurenelementen in der Haut führt. Diesen erhöhten Bedarf kann durch Aufnahme von Calcium, Magnesium, Zink, Jod, Kalium, Kupfer, Mangan, Chrom, Selen und Molybdän auch in Kombination mit Vitamin D, Bromelain und L-Glutaminsäure entgegengewirkt werden.

Die Auflistung der durch Stress hervorgerufenen Mangelerscheinungen sei hier nur beispielhaft genannt.

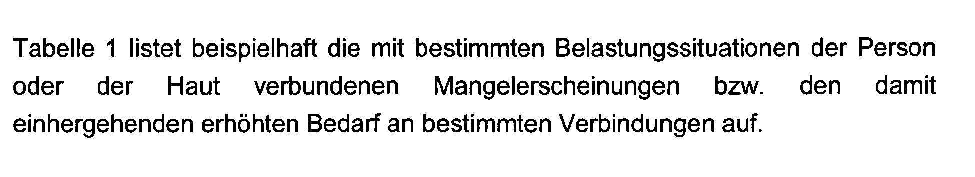

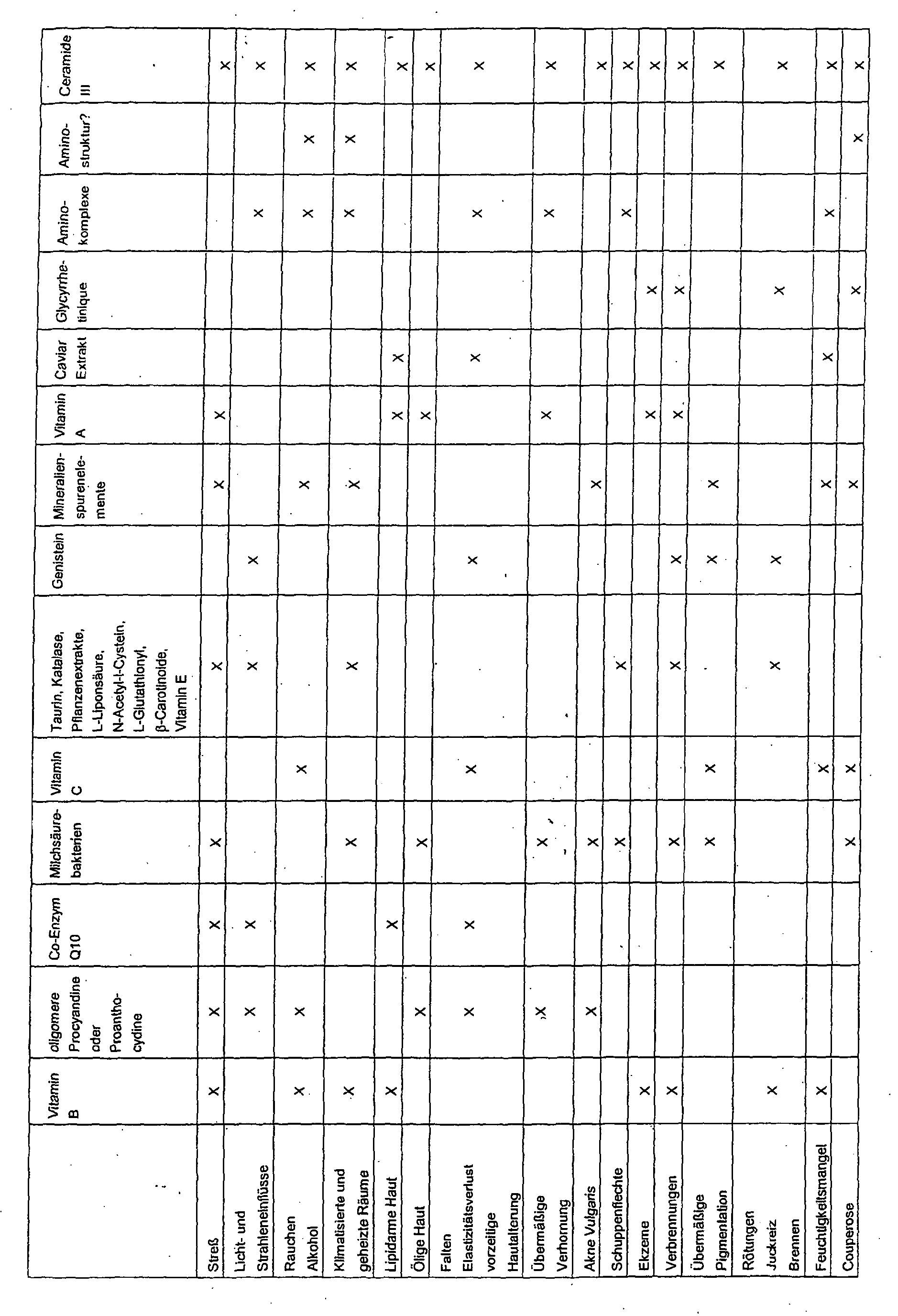

Es wurde nun festgestellt, dass die Aufnahme von Nahrungsergänzungspräparaten, welche die den mit bestimmten Hautsituationen korrelierenden Mangelerscheinungen entgegenwirkende Verbindungen enthalten, und eine gleichzeitige äußere Applikation von nährstoffhaltigen Verbindungen den Mangelsituationen der Haut effektiv entgegenwirken kann.

Die Nahrungsergänzungsmittel zur inneren Behandlung der Mangelsituation der Haut enthalten erfindungsgemäß wenigstens eine Substanz der Gruppe bestehend aus Vitamin A, Vitamin C, Vitamin E, Vitamin B1, Vitamin B2, Vitamin B3, Vitamin B5, Vitamin B6, Vitamin B12, Vitamin D, Vitamin E, Vitamin K, Folsäure, Biotin, Cholin, Inosit, para-Aminobenzoesäure, Co-Enzym Q10, L-Carnitin, Papain, alpha-Liponsäure, N-Acetyl-1-cystein, L-Glutathion, Carotinoide, Taurin, Katalase, Zink, Selen, Calcium, Kalium, Magnesium, Mangan, Chrom, Kupfer, Molybdän, Jod, Bromelain, L-Glutaminsäure, Bioflavonoid Komplexe, Rutin, Quercetin, Hesperidin, Isoflavone, Genistein, Daidzein, Glycitin, Niacin, Pflanzenextrakte der Gruppe bestehend aus Heidelbeeren, Traubenkerne, grüner Tee, Milchdistel, Ginko Biloba, Kuduz, Holunderbeeren, Hagebutte, rote Trauben, Zitronen, Kurkuma, Kiefernrinde, Acerola, mehrfach ungesättigte Fettsäure, Milchsäurebakterien, oligomere Procyandine und Proanthocydine.

Die zur äußeren Applikation vorgesehenen Zubereitungen weisen erfindungsgemäß wenigstens eine Verbindung der Gruppe bestehend aus Vitamin A, Vitamin C, Vitamin E, Vitamin B1, Vitamin B2, Vitamin B3, Vitamin B5, Vitamin B6, Vitamin B12, Vitamin D, Vitamin E, Vitamin K, Folsäure, Biotin, Cholin, Inosit, para-Aminobenzoesäure, Co-Enzym Q10, L-Carnitin, Papain, alpha-Liponsäure, N-Acetyl-1-cystein, L-Glutathion, Carotinoide, Taurin, Katalase, Zink, Selen, Calcium, Kalium, Magnesium, Mangan, Chrom, Kupfer, Molybdän, Jod, Bromelain, L-Glutaminsäure, Bioflavonoid Komplexe, Rutin, Quercetin, Hesperidin, Isoflavone, Genistein, Daidzein, Glycitin, Niacin, Creatinin, Geraniol, Limonen, Linalool, Hyaloronsäure, Mannitol, Glutamin, Allantoin, eine Aminosäure der Gruppe bestehend aus Glycin, Allanin, Arginin, Glutaminsäure, Asparaginsäure, Lysin, Leucin, Isoleucin, Phenylallanin, Histidin, Tyrosin, Prolin, oder Derivate der zuvor genannten Verbindungen, Pflanzenextrakte der Gruppe bestehend aus Heidelbeeren, Traubenkerne, grüner Tee, Milchdistel, Süßholzwurzel, Ginko Biloba, Kuduz, Holunderbeeren, Hagebutte, rote Trauben, Zitronen, Kurkuma, Kiefernrinde, Acerola, mehrfach ungesättigte Fettsäure, Milchsäurebakterien, Molkenprotein, oligomere Procyandine, Proanthocydine, und Caviar-Extrakt.

Die Zubereitungen zur äußeren Behandlung der Mangelsituation der Haut können darüber hinaus Hilfsstoffe wie Alkylenglycole, Panthenol, Alkohol, Bis-PEG-15 Methylether Dimeticon, Phenoxyethanol, Glycerin, Xanthan Gummi, Natriumacrylate, Parfüme, Titandioxid, Hexanoyl Dipeptid-3-Norleucinacetat, PEG-40 hydriertes Castoröl, Natriumcitrat, Ethylenhexylglycerin, Natriumsorbat, Methylparaben, Ethylparaben, Butylparaben, Butylphenyl Methylpropional, Caprate wie Propylenglycol Dicaprylate oder Dicaprate, Isodecylcitrat, Polysorbat 20, Polyacrylamid, C 13-14 Isoparafin, Zimtalkohol, Kupfergluconat, Diazolidinylharnstoff, Edta, lodopropynylbutylcarbanat, Mannitol, Tromethamin, Ethylhexylmethoxycinnamat, sowie für kosmetische Anwendungen typische Netzmittel und Emolgatoren.

Zur Verbesserung der Hautzugänglichkeit können erfindungsgemäß Wirkstoffe Liposomal gekapselt sein.

Hierbei hat sich insbesondere herausgestellt, dass die Aufnahme von Nahrungsergänzungspräparaten und die äußere Applikation von entsprechenden nährstoffhaltigen Verbindungen besonders effektiv ist, wenn die Nahrungsergänzungspräparate und die nährstoffhaltigen Verbindungen zur äußeren Applikation in weiten Bereichen hinsichtlich der in ihnen enthaltenen Verbindungen identisch sind.

Hierdurch wird eine gleichmäßige und dauerhafte Bereitstellung der von der Haut zur Beseitigung der Mangelsituation benötigten Nährstoffe durch die Nahrungsergänzungspräparate gewährleistet, wobei die äußere Anwendung zu einer schnellen Bereitstellung der für die Beseitigung der Mangelsituation benötigten Verbindungen in der Haut sorgt.

Hierzu ist es notwendig, dass die für die äußere Applikation eingesetzten nährstoffhaltigen Verbindungen hinsichtlich ihrer Zusammensetzung auf die Nahrungsergänzungspräparate abgestimmt sind und somit für jede zu behandelnde Person individuell zubereitet werden.

Die vorliegende Erfindung betrifft daher ebenfalls eine Vorrichtung zur Durchführung eines kosmetischen Verfahrens der zuvor beschriebenen Art, wobei die Vorrichtung einen Informationsträger sowie eine Ausgabeeinrichtung aufweist, wobei auf dem Informationsträger Informationen hinsichtlich der mit der jeweiligen festgestellten Hautsituation korrespondierenden Mangelsituation der Haut hinterlegt und mittels der Ausgabeeinrichtung abrufbar sind.

Hierdurch kann in einfacher Weise aufgrund der festgestellten Hautsituation eine Auswahl der aufzunehmenden Nahrungsergänzungspräparate sowie der äußerlich zu applizierenden nährstoffhaltigen Verbindungen getroffen werden.

In einer Ausgestaltung der erfindungsgemäßen Vorrichtung ist der Informationsträger ein elektronischer Datenträger, welcher mit einer Datenverarbeitungseinrichtung verbunden ist, welche wiederum mit einer Eingabeeinrichtung zur Eingabe der jeweils festgestellten Haut- und/oder Mangelsituation verbunden ist, wobei die Datenverarbeitungseinrichtung automatisch in Abhängigkeit der eingegebenen Haut- und/oder Mangelsituation Informationen über die zur Behebung der Mangelsituation der Haut geeigneten Narhungsergänzungsmittel und Verbindungen zur äußeren Applikation von dem Informationsträger abruft und mittels der Ausgabeeinrichtung ausgibt.

In einer weiteren Ausgestaltung einer zuvor beschriebenen Vorrichtung ist die Datenverarbeitungseinrichtung mit einer Einrichtung zur Ausgabe von Verbindungen zur äußeren Applikation verbunden, wobei die Einrichtung zur Ausgabe von Verbindungen zur äußeren Applikation Vorratsbehälter für die Verbindungen zur äußeren Applikation aufweist und die Ausgabevorrichtung in Abhängigkeit der durch die Datenverarbeitungseinrichtung vom Informationsträger vermittelten Informationen automatisch Verbindungen zur äußeren Applikation aus dem Vorratsbehältern entnimmt und zur äußeren Applikation bereitstellt.

Eine solche Vorrichtung kann darüber hinaus eine Einrichtung zum Mischen der einzelnen Verbindungen zur äußeren Applikation vor einer Bereitstellung aufweisen.

Durch die erfindungsgemäße Vorrichtung wird es dem Anwender des erfindungsgemäßen Verfahrens, bei dem in der Regel von einem Kosmetiker oder einer Kosmetikerin auszugehen ist, zuverlässig und einfach möglich, eine in Abhängigkeit der festgestellten Hautsituation geeignete kosmetische Behandlung festzulegen. Besonders vorteilhaft bei der zuvor beschriebenen Ausgestaltung der erfindungsgemäßen Vorrichtung ist dabei, dass automatisch eine für jede zu behandelnde Person individuell zubereitete Mischung an nährstoffhaltigen Verbindungen zur äußeren Applikation bereitgestellt wird, so dass die aufgetretenen Mangelsituationen der Haut spezifisch und individuell behandelt werden können.

## Patentansprüche

1. Kosmetisches Verfahren zur Beeinflussung der Haut, aufweisend die Verfahrensschritte:
- Feststellen der Hautsituation durch Bestimmung des Pflegezustandes der Haut, des Alters und/oder der Energie- und Belastungssituation der Person;
- Feststellen der Mangelsituation der Haut in Abhängigkeit der zuvor festgestellten Hautsituation;
- Festlegen eines Behandlungsplans in Abhängigkeit der zuvor festgestellten Mangelsituation der Haut, umfassend die Aufnahme von Nahrungsergänzungspräparaten zur inneren Behandlung der festgestellten Mangelsituation der Haut und eine äußere Applikation von nährstoffhaltigen Verbindungen zur gleichzeitigen ergänzenden äußeren Behandlung der festgestellten Mangelsituation der Haut.

2. Verfahren gemäß Anspruch 1, wobei als Nahrungsergänzungsmittel zur inneren Behandlung der Mangelsituation der Haut wenigstens eine Substanz der Gruppe bestehend aus Vitamin A, Vitamin C, Vitamin E, Vitamin B1, Vitamin B2, Vitamin B3, Vitamin B5, Vitamin B6, Vitamin B12, Vitamin D, Vitamin E, Vitamin K, Folsäure, Biotin, Cholin, Inosit, para-Aminobenzoesäure, Co-Enzym Q10, L-Carnitin, Papain, alpha-Liponsäure, N-Acetyl-1-cystein, L-Glutathion, Carotinoide, Taurin, Katalase, Zink, Selen, Calcium, Kalium, Magnesium, Mangan, Chrom, Kupfer, Molybdän, Jod, Bromelain, L-Glutaminsäure, Bioflavonoid Komplexe, Rutin, Quercetin, Hesperidin, Isoflavone, Genistein, Daidzein, Glycitin, Niacin, Pflanzenextrakte der Gruppe bestehend aus Heidelbeeren, Traubenkerne, grüner Tee, Milchdistel, Ginko Biloba, Kuduz, Holunderbeeren, Hagebutte, rote Trauben, Zitronen, Kurkuma, Kiefernrinde, Acerola, mehrfach ungesättigte Fettsäure, Milchsäurebakterien, oligomere Procyandine und Proanthocydine aufgenommen wird.

3. Verfahren gemäß Anspruch 1, wobei bei der Applikation zur äußeren Behandlung der Mangelsituation der Haut wenigstens eine Verbindung der Gruppe bestehend aus Vitamin A, Vitamin C, Vitamin E, Vitamin B1, Vitamin B2, Vitamin B3, Vitamin B5, Vitamin B6, Vitamin B12, Vitamin D, Vitamin E, Vitamin K, Folsäure, Biotin, Cholin, Inosit, para-Aminobenzoesäure, Co-Enzym Q10, L-Carnitin, Papain, alpha-Liponsäure, N-Acetyl-I-cystein, L-Glutathion, Carotinoide, Taurin, Katalase, Zink, Selen, Calcium, Kalium, Magnesium, Mangan, Chrom, Kupfer, Molybdän, Jod, Bromelain, L-Glutaminsäure, Bioflavonoid Komplexe, Rutin, Quercetin, Hesperidin, Isoflavone, Genistein, Daidzein, Glycitin, Niacin, Creatinin, Geraniol, Limonen, Linalool, Hyaloronsäure, Mannitol, Glutamin, Allantoin, eine Aminosäure der Gruppe bestehend aus Glycin, Allanin, Arginin, Glutaminsäure, Asparaginsäure, Lysin, Leucin, Isoleucin, Phenylallanin, Histidin, Tyrosin, Prolin, oder Derivate der zuvor genannten Verbindungen, Pflanzenextrakte der Gruppe bestehend aus Heidelbeeren, Traubenkerne, grüner Tee, Milchdistel, Süßholzwurzel, Ginko Biloba, Kuduz, Holunderbeeren, Hagebutte, rote Trauben, Zitronen, Kurkuma, Kiefernrinde, Acerola, mehrfach ungesättigte Fettsäure, Milchsäurebakterien, Molkenprotein, oligomere Procyandine, Proanthocydine, und Caviar-Extrakt auf die Haut appliziert wird.

4. Verfahren gemäß einem der vorhergegangenen Ansprüche, wobei bei der Applikation zur äußeren Behandlung der Mangelsituation der Haut wenigstens die Verbindungen auf die Haut appliziert werden, die als Nahrungsergänzungsmittel zur inneren Behandlung der Mangelsituation der Haut aufgenommen wurden.

5. Vorrichtung zur Durchführung eines kosmetischen Verfahrens nach einem der Ansprüche 1 bis 4, aufweisend einen Informationsträger sowie eine Ausgabeeinrichtung, wobei auf dem Informationsträger Informationen hinsichtlich der mit der jeweilig festgestellten Hautsituation korrespondierenden Mangelsituation hinterlegt und mittels der Ausgabeeinrichtung abrufbar sind.

6. Vorrichtung gemäß Anspruch 5, wobei auf dem Informationsträger Informationen hinsichtlich der mit einem jeweiligen Nahrungsergänzungsmittel und/oder der äußerlich applizierten Verbindung zu behebenden Mangelsituation der Haut hinterlegt und mittels der Ausgabeeinrichtung abrufbar sind.

7. Vorrichtung gemäß einem der Ansprüche 5 und 6, wobei der Informationsträger ein elektronischer Datenträger ist, welcher mit einer Datenverarbeitungseinrichtung verbunden ist, welche mit einer Eingabeeinrichtung zur Eingabe der jeweils festgestellten Haut- und/oder Mangelsituation verbunden ist, wobei die Datenverarbeitungseinrichtung automatisch in Abhängigkeit der eingegebenen Haut- und/oder Mangelsituation Informationen über die zur Behebung der Mangelsituation der Haut geeigneten Nahrungsergänzungsmittel und Verbindungen zur äußeren Applikation von dem Informationsträger abruft und mittels der Ausgabeeinrichtung ausgibt.

8. Vorrichtung gemäß Anspruch 7, wobei die Datenverarbeitungseinrichtung mit einer Einrichtung zur Ausgabe von Verbindungen zur äußeren Applikation verbunden ist, wobei die Einrichtung zur Ausgabe von Verbindungen zur äußeren Applikation Vorratsbehälter für die Verbindungen zur äußeren Applikation aufweist und die Ausgabevorrichtung in Abhängigkeit der durch die Datenverarbeitungseinrichtung vom Informationsträger ermittelten Informationen automatisch Verbindungen zur äußeren Applikation aus den Vorratsbehältern entnimmt und zur äußeren Applikation bereitstellt.

9. Vorrichtung gemäß Anspruch 8, wobei die Einrichtung zur Ausgabe der Verbindungen zur äußeren Applikation eine Einrichtung zum Mischen der einzelnen Verbindungen zur äußeren Applikation vor einer Bereitstellung aufweist.
